(19) Europäisches Patentamt
European Patent Office
Office européen des brevets

(11) Veröffentlichungsnummer: **0 324 336 B2**

(12) **NEUE EUROPÄISCHE PATENTSCHRIFT**

(45) Veröffentlichungstag der neue Patentschrift: **08.11.95**

(51) Int. Cl.[6]: **C07D 207/34**, //C07C255/00

(21) Anmeldenummer: **89100004.4**

(22) Anmeldetag: **02.01.89**

(54) **Verfahren zur Herstellung von 3-Cyano-4-aryl-pyrrolen.**

(30) Priorität: **09.01.88 DE 3800387**

(43) Veröffentlichungstag der Anmeldung:
**19.07.89 Patentblatt 89/29**

(45) Bekanntmachung des Hinweises auf die Patenterteilung:
**18.03.92 Patentblatt 92/12**

(45) Bekanntmachung des Hinweises auf die Entsheidung über den Einspruch:
**08.11.95 Patentblatt 95/45**

(84) Benannte Vertragsstaaten:
**BE CH DE FR GB IT LI NL**

(56) Entgegenhaltungen:
**EP-A- 378 046**
**EP-A- 0 293 711**
**DE-A- 3 601 285**
**US-A- 4 965 363**

**Tetrahedron Letters, 52, pp. 5337-5340 (1972), van Leusen et al.**

**Houben-Weyl, "Methoden der organ. Chemie", Bd. 8, Sauerstoff-III, S. 651 (1952)**

**Morrison, Boyd, "Organic Chemistry", pp. 753-755 (1992)**

**Pine, Hendrickson, Cram, Hammond, "Organische Chemie, S. 7 (1987)**

**Synthesis, 1987, pp. 566-568**

**Weissberger, "The chemistry of heterocyclic compounds, Pyrroles", part I, 1990, p. 203, 645**

**Houben-Weyl, "Methoden der organischen Chemie", Band X/3, Stickstoff-Verbindungen I, Teil 3, p. 665, p. 662**

**Chemische Berichte, 104, 3816, 3817, 3824 (1971)**

(73) Patentinhaber: **BAYER AG**

**D-51368 Leverkusen (DE)**

(72) Erfinder: **Wollweber, Detlef, Dr.**
**Paul-Ehrlich-Strasse 17**
**D-5600 Wuppertal 1 (DE)**



Rank Xerox (UK) Business Services
(3.10/3.09/3.3.3)

## Beschreibung

Die Erfindung betrifft ein neues Verfahren zur Herstellung von 3-Cyano-4-aryl-pyrrolen, welche als Fungizide bekannt sind.

Es ist bekannt, daß man 3-Cyano-4-aryl-pyrrole erhält, wenn man Zimtsäurenitrile in Gegenwart von Natriumhydrid mit p-Toluolsulfonylmethylisocyanid umsetzt (vgl. DE-OS 29 27 480). Dieses Verfahren bringt jedoch mit ca 35% Ausbeute nur unbefriedigende Ergebnisse. Nachteilig ist außerdem, daß die so erhältlichen Verbindungen aufwendig gereinigt werden müssen (vgl. J6-1030-571). Schließlich ist das als Reagenz verwendete Natriumhydrid für technische Synthesen wegen der hohen Hydrolyseanfälligkeit und der damit verbundenen Brandgefahr des bei der Hydrolyse freigesetzten gasförmigen Wasserstoffes ungeeignet.

Weiterhin ist bekannt, daß man 3-Cyano-4-aryl-pyrrole auch erhält, wenn man α-Cyanozimtsäureester oder-säuren in Gegenwart von Basen und gegebenenfalls in Gegenwart von Kupfer-(II)-Salzen mit p-Toluolsulfonylmethylisocyanid umsetzt (vgl. J6-1030-571 oder J6-1200-984 sowie US-PS 4 680 413). Nachteilig bei diesem Verfahren ist, daß die als Ausgangsverbindungen benötigten α-Cyanozimtsäureester erst in einem aufwendigen Verfahren hergestellt werden müssen (vgl. J. chem. Soc. 1961, 683).

Außerdem ist bekannt, daß man 3-Cyano-4-aryl-pyrrole auch erhält, wenn man α-substituierte Zimtsäurenitrile in Gegenwart von Natriumhydrid mit Isocyanoessigsäureethylester cyclisiert, die so erhäldichen Pyrrol-2-carbonsäureester mit Basen verseift und anschließend thermisch decarboxyliert (vgl. JP 59/212 468). Der technischen Anwendbarkeit dieses Verfahrens stehen wiederum die ungünstigen Eigenschaften des Natriumhydrids im Wege. Auch die Ausbeuten des Cyclisierungsschrittes sind mit 44 % nicht zufriedenstellend.

Weiterhin ist bekannt, daß man 3-Cyano-4-aryl-pyrrole erhält, wenn man Phenacylamin-Derivate mit geeignet substituierten Acrylnitril-Derivaten umsetzt (vgl. EP 174 910). Die als Ausgangsverbindungen erforderlichen Phenacylaminderivate sind jedoch nur über eine aufwendige, vielstufige Synthese zugänglich, in deren Vertauf unter anderem auch der unerfreuliche Einsatz von Cyaniden notwendig ist.

Weiterhin ist bekannt, daß man 3-Cyano-4-aryl-pyrrole erhält, wenn man die entsprechenden 3-Trifluormethyl-4-aryl-pyrrole mit Ammoniak bei erhöhter Temperatur und erhöhtem Druck umsetzt (vgl. EP 182 736). Auch bei diesem Verfahren sind jedoch die als Ausgangsprodukte erforderlichen 3-Trifluormethyl-4-arylpyrrole nur über einen aufwendigen, mehrstufigen Weg zugänglich, wobei die Verwendung von feuchtigkeitsempfindlichen "Wittig-Reagenzien" und kostspieligem Trifluoracetanhydrid im Verlauf dieser mehrstufigen Synthese die technische Durchführbarkeit zusätzlich erschwert.

Schließlich ist bekannt, daß man 3-Cyano-4-aryl-pyrrole erhält, wenn man 3-Cyano-4-aryl-Δ$^2$-pyrroline in Gegenwartvon Cu-II-Salzen oder Eisen-III-salzen oxidiert (vgl. EP 183 217). Auch bei diesem letzten Verfahren ist die Herstellung der erforderlichen Ausgangsverbindungen mehrstufig und technisch aufwendig.

Es wurde gefunden, daß man 3-Cyano-4-aryl-pyrrole der allgemeinen Formel (I),

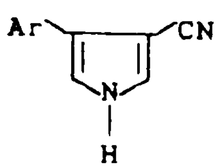

(I)

in welcher
Ar für jeweils gegebenenfalls substituiertes Heteroaryl oder Aryl steht,
erhält, wenn man α-Cyanozimtsäureamide der Formel (II),

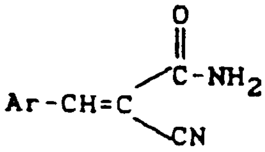

(II)

in welcher
Ar die oben angegebene Bedeutung hat,
mit Sulfonylmethylisocyaniden der Formel (III),

$R-SO_2-CH_2-NC$ (III)

in welcher
R für Alkyl, für gegebenenfalls substituiertes Cycloalkyl oder für gegebenenfalls substituiertes Aryl steht, in Gegenwart einer Base und in Gegenwart eines Verdünnungsmittels umsetzt.

Es ist als ausgesprochen überraschend anzusehen, daß das erfindungsgemäße Verfahren die gewünschten 3-Cyano-4-aryl-pyrrole der allgemeinen Formel (I) in guten Ausbeuten zwischen 91 und 94 % und in hoher Reinheit liefert, da nach dem, was aus dem Stand der Technik bekannt war, beide möglichen Reaktionswege, die zu den Endprodukten führen könnten, nicht als naheliegend angesehen werden konnten. Es war einerseits nicht zu erwarten, daß die Verseifung einer Amidgruppierung und die anschließende Decarboxylierung unter so milden Bedingungen ablaufen würde (vgl. z. B. J. org. Chem. 46, 5351 - 5353 [1981]). Die alternative direkte Abspaltung einer Carbonamidgruppe unter Freisetzung von Isocyansäure andererseits ist in der Literatur bisher nicht bekannt.

Zu erwarten gewesen wäre aus dem Stand der Technik vielmehr, daß die intermediär auftretenden Zwischenprodukte der Formel (IV),

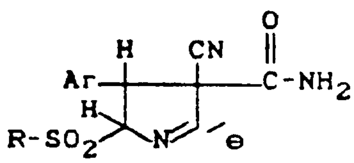

(IV)

in welcher
R und Ar die oben angegebene Bedeutung haben,
sich durch Abspaltung von Sulfinaten zu den 3-H-Pyrrolen der Formel (V),

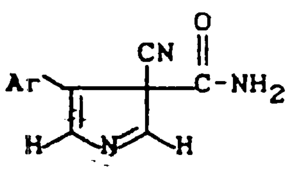

(V)

in welcher
Ar die oben angegebene Bedeutung hat,
stabilisieren würden, aus welcher die Säureamidgruppierung nicht ohne weiteres selektiv abspaltbarwäre (vgl. US-PS 4 680 413).

Die erfindungsgemäße Verfahren besitzt gegenüber dem nächtsliegenden Stand der Technik (US-PS 4 680 413) den Vorteil, daß die als Ausgangsprodukte verwendeten α-Cyanozimtsäureamide der Formel (II) unter wesentlich milderen Bedingungen herstellbar sind als beispielsweise die entsprechenden α-Cyanozimtsaureester (vgl. z. B. J. chem. Soc. 1961, 683) und darüberhinaus wesentlich unempfindlicher bei höheren Temperaturen sind, wo die entsprechenden α-Cyanozimtsäureester oder die entsprechenden freien Säuren bereits zu vorzeitiger Decarboxylierung neigen.

Die mit Hilfe des erfindungsgemäßen Verfahrens erhältlichen 3-Cyano-4-aryl-pyrrole sind durch die Formel (I) allgemein definiert.

Bevorzugt herstellbar sind Verbindungen der Formel (I), bei welchen Ar für jeweils gegebenenfalls einfach bis mehrfach, gleich oder verschieden durch Halogen und geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl steht, wobei als Substituenten in Frage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen, jeweils geradkettiges oder versweigtes Halogenalkyl,

Halogenalkoxy oder Halogenalkylthio mit jeweils 1 bis 4 Kohlenstoffatomen und 1 bis 9 gleichen oder verschiedenen Halogenatomen sowie zweifach verknüpftes, gegebenenfalls durch Fluor substituiertes Dioxyalkylen mit 1 oder 2 Kohlenstoffatomen.

Besonders bevorzugt herstellbar sind Verbindungen der Formel (I), bei welchen Ar für jeweils gegebenenfalls einfach bis dreifach, gleich oder verschieden durch Fluor, Chlor, Brom, Methyl und Ethyl substituiertes 2-Pyridyl, 4-Pyridyl, 2-Furyl oder 2-Thienyl steht oder für gegebenenfalls ein-bis dreifach, gleich oder verschieden substituiertes Phenyl steht, wobei als Substituenten infrage kommen: Fluor, Chlor, Brom, Methyl, Ethyl, n- oder i-Propyl, Methoxy, Ethoxy, Methylthio, Trifluormethyl, Trifluormethoxy, Trifluonnethylthio, Cyano, Nitro, Dioxymethylen und Dioxydifluormethylen.

Ganz besonders bevorzugt herstellbar sind Verbindungen der Formel (I), bei welchen Ar für gegebenenfalls einfach bis dreifach, gleich oder verschieden substituiertes Phenyl steht, wobei als Substituenten infrage kommen: Fluor Chlor, Brom, Methyl, Ethyl, Methoxy, Ethoxy, Methylthio, Trifluormethyl, Trifluormethoxy, Trifluormethylthio, Nitro und Dioxydifluormethylen.

Verwendet man beispielsweise 2-(2,3-Dichlorphenyl-methyliden)-cyanessigsäureamid und p-Toluolsulfonylmethylisocyanid als Ausgangsstoffe, so läßt sich der Reaktionsablauf des erfindungsgemäßen Verfahrens durch das folgende Formelschema darstellen:

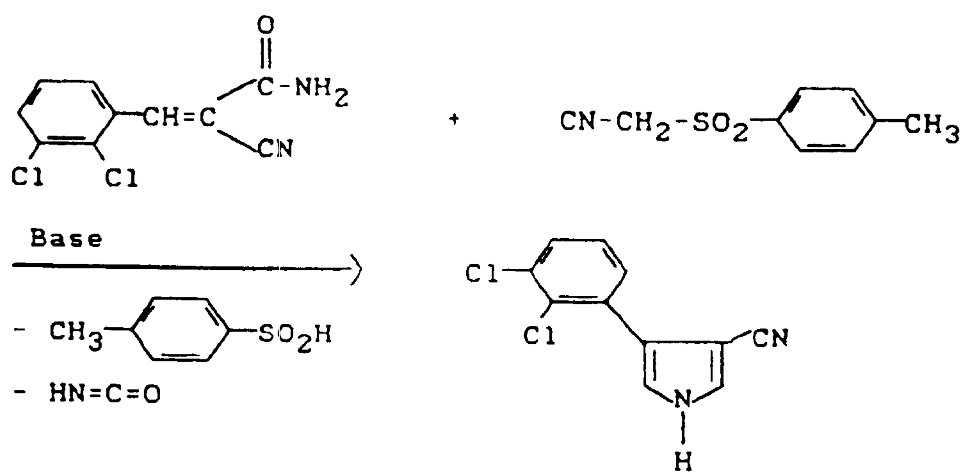


Die zur Durchführung des erfindungsgemäßen Verfahrens als Ausgangsstoffe benötigten α-Cyanozimtsäureamide sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diese Substituenten genannt wurden.

Die α-Cyanozimtsäureamide der Formel (II) sind bekannt oder erhältlich in Analogie zu bekannten Verfahren (vgl. z. B. J. chem. Soc. 1961, 683), beispielsweise wenn man Aldehyde der Formel (VI),

Ar-CHO (VI)

in welcher
Ar die oben angegebene Bedeutung hat,
mit α-Cyanacetamid der Formel (VII)

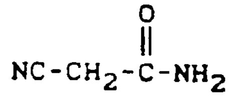

(VII)

gegebenenfalls in Gegenwart eines Verdünnungsmittels wie beispielsweise Ethanol und gegebenenfalls in Gegenwart einer Base wie beispielsweise Kaliumhydroxid oder Piperidin bei Temperaturen zwischen +20 °C und +150 °C kondensiert.

Die Aldehyde der Formel (VI) und das α-Cyanacetamid der Formel (VII) sind allgemein bekannte Verbindungen der organischen Chemie oder erhältlich in Analogie zu bekannten Verfahren.

Die zur Durchführung des erfindungsgemäßen Verfahrens weiterhin als Ausgangsstoffe benötigten Sulfonylmethylisocyanide sind durch die Formel (III) allgemein definiert. In dieser Formel (III) steht R vorzugsweise für geradkettiges oder verzweigtes Alkyl mit 1 bis 4 Kohlenstoffatomen, für gegebenenfalls einfach bis mehrfach, durch geradkettiges oder verzweigtes Alkyl mit 1 bis 4 Kohlenstoffatomen und/oder Halogen substituiertes Cycloalkyl mit 3 bis 7 Kohlenstoffatomen oder für gegebenenfalls einfach bis mehrfach, gleich oder verschieden substituiertes Aryl mit 6 bis 10 Kohlenstoffatomen, wobei als Substituenten in Frage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen. R steht besonders bevorzugt für Methyl, Cyclohexyl oder für jeweils gegebenenfalls ein- bis dreifach, gleich oder verschieden substituiertes Phenyl oder Naphthyl, wobei als Substituenten jeweils in Frage kommen: Fluor, Chlor, Brom, Cyano, Nitro, Methyl, Ethyl, n- oder i-Propyl, n-, i-, s- oder 5-Butyl, Methoxy, Ethoxy, n- oder i-Propoxy, Methylthio, Trifluormethyl, Trifluormethoxy, Trifluormethylthio.

Die Sulfonylmethylisocyanide der Formel (III) sind bekannt oder können in Analogie zu bekannten Verfahren erhalten werden (vgl. z. B. DE-OS 36 01 285; US-PS 4 680 413; Tetrahedron Lett. 1972, 2367 - 2368; J. Org. Chem. 42, 1153 - 1159 [1977]; Synthesis 1985, 400 - 402; Organic Syntheses 57, 102 - 106 [1977]).

Als Verdünnungsmittel zur Durchführung des erfindungsgemäßen Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, oder Alkohole, wie Methanol, Ethanol, Propanol oder Butanol.

Das erfindungsgemäße Verfahren wird in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat.

Die Reaktionstemperaturen können bei der Durchführung des erfindungsgemäßen Verfahrens in einem größeren Bereich variiert werden. Im allgemeinen arbeitet man bei Temperaturen zwischen -30 °C und +100 °C, vorzugsweise bei Temperaturen zwischen -10 °C und +40 °C.

Zur Durchführung des erfindungsgemäßen Verfahrens setzt man pro Mol an α-Cyanozimtsäureamid der Formel (II) im allgemeinen 1.0 bis 2.0 Mol, vorzugsweise 1.0 bis 1.3 Mol an Sulfonylmethylisocyanid der Formel (III) und 1.0 bis 6.0 Mol, vorzugsweise 1.0 bis 3.0 Mol an Base ein. Dabei geht man so vor, daß zunächst das α-Cyanozimtsäureamid der Formel (II) zusammen mit der Base in einem geeigneten Lösungsmittel vorgelegt wird, anschließend das Sulfonylmethylisocyanid der Formel (III) in einem geeigneten Lösungsmittel gelöst tropfenweise dazu gegeben wird, danach für einige Stunden bei der erforderlichen Reaktionstemperatur gerührt wird, organisches Lösungsmittel im Vakuum entfernt wird und der Rückstand mit Wasser behandelt wird, wobei die gewünschten 3-Cyano-4-aryl-pyrrole der Formel (I) als Niederschlag anfallen und alle Nebenprodukte in Lösung bleiben. Durch Filtration und Trocknung erhält man Produkte von hoher Ausbeute und Reinheit.

Die so erhältlichen 3-Cyano-4-aryl-pyrrole sind bekannte Fungizide (vgl. z. B. EP 236 272) oder wichtige Zwischenprodukte für Fungizide (vgl. z. B. US-PS 4 680 413 oder DE-OS 29 27 480 oder EP 182 737).

Herstellungsbeispiele

Beispiel 1

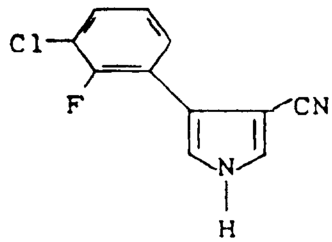

Zu 6,75g (0.03 Mol) 2-(2-Fluor-3-chlorphenyl-methyliden)-cyanessigsäureamid in 40 ml Ethanol gibt man bei 0 °C bis 5 °C eine Lösung von 0,95 g (0.041 Mol) Natrium in 20 ml Ethanol und anschließend tropfenweise unter Rühren ebenfalls bei 0 °C bis 5 °C eine Lösung aus 7,0 g (0.036 Mol) p-Toluolsulfonylmethylisocyanid in 50 ml Dichlormethan, rührt nach beendeter Zugabe 1 Stunde bei 0 °C und 3 Stunden bei Raumtemperatur, gibt dann 30 ml Wasser zu, stellt mit 1 normaler Salzsäure auf pH 3 ein, entfernt organisches Lösungsmittel im Vakuum, gibt nochmals 60 ml Wasser zu, rührt 30 Minuten bei Raumtemperatur, saugt ab und trocknet den so erhaltenen Feststoff.

Man erhält 6,2 g (94 % der Theorie) an 3-Cyano-4-(2-fluor-3-chlorphenyl)-pyrrol vom Schmelzpunkt 180 °C - 181 °C.

Herstellung der Ausgangsverbindung

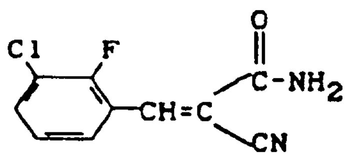

Zu 98,3 g (0.62 Mol) 2-Fluor-3-chlorbenzaldehyd (vgl. DE-OS 31 29 277) in 500 ml Ethanol gibt man nacheinander 52,3 g (0.62 Mol) Cyanacetamid und 3 g (0.05 Mol) Kaliumhydroxid und rührt anschließend 15 Stunden bei Raumtemperatur. Der ausgefallene Niederschlag wird abgesaugt und getrocknet. Man erhält 91,6 g (66 % der Theorie) an 2-(2-Fluor-3-chlorphenylmethyliden)-cyanacetamid vom Schmelzpunkt 160 °C - 162 °C.

In entsprechender Weise und gemäß den allgemeinen Angaben zur Herstellung erhält man die folgenden 3-Cyan-4-aryl-pyrrole der allgerneinen Formel (I):

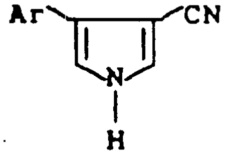

(I)

| Bsp.-Nr. | Ar | Ausbeute | Schmelzpunkt (°C) |
|---|---|---|---|
| 2 | 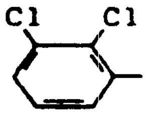 | 93 % | 139 - 141 |
| 3 | 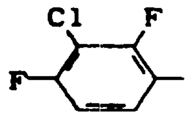 | 91 % | 218 - 219 |

## Patentansprüche

**1.** Verfahren zur Herstellung von 3-Cyano-4-arylpyrrolen der allgemeinen Formel (I),

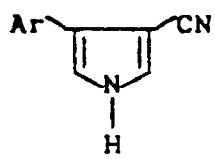
(I)

in welcher
Ar für jeweils gegebenenfalls substituiertes Heteroaryl oder Aryl steht,
dadurch gekennzeichnet, daß man $\alpha$-Cyanozimtsäureamide der Formel (II),

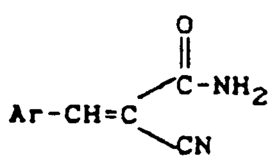
(II)

in welcher
Ar die oben angegebene Bedeutung hat,
mit Sulfonylmethylisocyanicien der Formel (III).

$R-SO_2-CH_2-NC$ (III)

in welcher
R für Alkyl, für gegebenenfalls substituiertes Cycloalkyl oder für gegebenenfalls substituiertes Aryl steht, in Gegenwart einer Base und in Gegenwart eines inerten organischen Lösungsmittels umsetzt.

**2.** Verfahren zur Herstellung von 3-Cyano-4-arylpyrrolen gemäß Anspruch 1, wobei in der Formel (I) Ar für jeweils gegebenenfalls einfach bis mehrfach, gleich oder verschieden durch Halogen und geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl steht, wobei als Substituenten in Frage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen sowie zweifach verknüpftes, gegebenenfalls durch Fluor substituiertes Dioxyalkylen mit 1 oder 2 Kohlenstoffatomen.

**3.** Verfahren zur Herstellung von 3-Cyano-4-arylpyrrolen gemäß Anspruch 1, wobei in der Formel (I) Ar für jeweils gegebenenfalls einfach bis dreifach, gleich oder verschieden durch Fluor, Chlor, Brom, Methyl und Ethyl substituiertes 2-Pyridyl, 4-Pyridyl, 2-Furyl oder 2-Thienyl steht oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl. Methoxy. Ethoxy, Methylthio, Trifluormethyl, Trifluormethoxy, Trifluormethylthio, Cyano, Nitro, Dioxymethylen und Dioxydifluormethylen.

**4.** Verfahren zur Herstellung von 3-Cyano-4-arylpyrrolen gemäß Anspruch 1, wobei das Verfahren bei Temperaturen zwischen -30 °C und +100 °C durchgeführt wird.

**5.** Verfahren zur Herstellung von 3-Cyano-4-arylpyrrolen gemäß Anspruch 4, wobei das Verfahren bei Temperaturen zwischen -10 °C und +40 °C durchgeführt wird.

**6.** Verfahren zur Herstellung von 3-Cyano-4-arylpyrrolen gemäß Anspruch 1, wobei man pro Mol α-Cyanozimtsäureamid der Formel (II) 1,0 bis 2,0 Mol Sulfonylmethylisocyanid der Formel (III) und 1,0 bis 6,0 Mol Base einsetzt.

**7.** Verfahren zur Herstellung von 3-Cyano-4-arylpyrrolen gemäß Anspruch 6, wobei man pro Mol α-Cyanozimtsäureamid der Formel (II) 1,0 bis 1,3 Mol an Sulfonylmethylisocyanid der Formel (III) und 1,0 bis 3,0 Mol Base einsetzt.

## Claims

**1.** Process for the preparation of 3-cyano-4-arylpyrroles of the general formula (I)

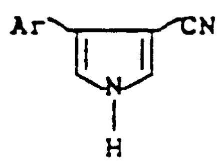

(I)

in which

Ar represents in each case optionally substituted heteroaryl or aryl,

characterised in that α-cyanocinnamamides of the formula (II)

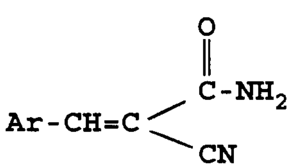

(II)

in which

Ar has the abovementioned meaning,

are reacted with sulphonylmethylisocyanides of the formula (III)

$R-SO_2-CH_2-NC$ (III)

in which

R represents alkyl, optionally substituted cycloalkyl or optionally substituted aryl,

in the presence of a base and in the presence of an inert organic solvent.

**2.** Process for the preparation of 3-cyano-4-arylpyrroles according to Claim 1, wherein, in the formula (I),

Ar represents pyridyl, furyl or thienyl, each of which is optionally monosubstituted or polysubstituted by identical or different substituents from the series comprising halogen and straight-chain or branched alkyl having 1 to 4 carbon atoms, or represents phenyl which is optionally monosubstituted or polysubstituted by identical or different substituents, suitable substituents being: halogen, cyano, nitro, in each case straight-chain or branched alkyl, alkoxy, alkylthio or alkoxycarbonyl, each having 1 to 4 carbon atoms, in each case straight-chain or branched halogenoalkyl, halogenoalkoxy or halogenoalkylthio, each having 1 to 4 carbon atoms and 1 to 9 identical or different halogen atoms, and double-linked dioxyalkylene having 1 or 2 carbon atoms which is optionally substituted by fluorine.

**3.** Process for the preparation of 3-cyano-4-arylpyrroles according to Claim 1, wherein, in the formula (I),
Ar represents 2-pyridyl, 4-pyridyl, 2-furyl or 2-thienyl, each of which is optionally monosubstituted, disubstituted or trisubstituted by identical or different substituents from the series comprising fluorine, chlorine, bromine, methyl and ethyl, or represents phenyl which is optionally monosubstituted, disubstituted or trisubstituted by identical or different substituents, suitable substituents being: fluorine, chlorine, bromine, methyl, ethyl, n- or i-propyl, methoxy, ethoxy, methylthio, trifluoromethyl, trifluoromethoxy, trifluoromethylthio, cyano, nitro, dioxymethylene and dioxydifluoromethylene.

**4.** Process for the preparation of 3-cyano-4-arylpyrroles according to Claim 1, wherein the process is carried out at temperatures between -30 °C and +100 °C.

**5.** Process for the preparation of 3-cyano-4-arylpyrroles according to Claim 4, wherein the process is carried out at temperatures between -10 °C and +40 °C.

**6.** Process for the preparation of 3-cyano-4-arylpyrroles according to Claim 1, wherein 1.0 to 2.0 mol of sulphonylmethylisocyanide of the formula (III) and 1.0 to 6.0 mol of base are employed per mole of $\alpha$-cyanocinnamamide of the formula (II).

**7.** Process for the preparation of 3-cyano-4-arylpyrroles according to Claim 6, wherein 1.0 to 1.3 mol of sulphonylmethylisocyanide of the formula (III) and 1.0 to 3.0 mol of base are employed per mole of $\alpha$-cyanocinnamamide of the formula (II).

**Revendications**

**1.** Procédé pour la préparation de 3-cyano-4-arylpyrroles répondant à la foule générale (I),

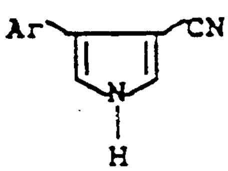

(I)

dans laquelle
Ar représente un groupe hétéroaryle ou un groupe aryle, chacun étant éventuellement substitué, **caractérisé en ce qu'**on fait réagir des amides de l'acide $\alpha$-cyanocinnamique de formule (II)

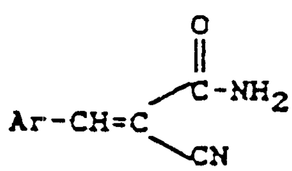

(II)

dans laquelle
Ar a la signification indiquée ci-dessus,

avec des isocyanures de sulfonylméthyle de formule (III),

$R-SO_2-CH_2-NC$ (III)

dans laquelle

R représente un groupe alkyle, un groupe cycloalkyle éventuellement substitué ou encore un groupe aryle éventuellement substitué,

en présence d'une base et en présence d'un solvant organique inerte.

**2.** Procédé pour la préparation de 3-cyano-4-arylpyrroles selon la revendication 1, dans lequel, dans la formule (I)

Ar représente un groupe pyridyle, un groupe furyle ou un groupe thiényle, chacun étant éventuellement substitué de une à plusieurs fois, de manière identique ou différente, par un atome d'halogène et par un groupe alkyle à chaîne droite ou ramifiée contenant de 1 à 4 atomes de carbone, ou encore un groupe phényle éventuellement substitué de une à plusieurs fois, de manière identique ou différente, les substituants entrant en ligne de compte étant : un atome d'halogène, un groupe cyano, un groupe nitro, un groupe alkyle, un groupe alcoxy, un groupe alkylthio ou un groupe alcoxycarbonyle contenant chacun de 1 à 4 atomes de carbone, chacun de ces groupes étant à chaîne droite ou à chaîne ramifiée, un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou à chaîne ramifiée, ainsi ou'un groupe dioxyalkylène à deux liaisons, éventuellement substitué par un atome de fluor, contenant 1 ou 2 atomes de carbone.

**3.** Procédé pour la préparation de 3-cyano-4-arylpyrroles selon la revendication 1, dans lequel, dans la formule (I)

Ar représente un groupe 2-pyridyle, un groupe 4-pyridyle, un groupe 2-furyle ou un groupe 2-thiényle, chacun de ces groupes étant éventuellement substitué de une à trois fois, de manière identique ou différente, par un atome de fluor, un atome de chlore, un atome de brome, un groupe méthyle et un groupe éthyle, ou représente un groupe phényle éventuellement substitué de une à trois fois, de manière identique ou différente, les substituants entrant en ligne de compte étant: un atone de fluor, un atome de chlore, un atome de brome, un groupe méthyle, un groupe éthyle, un groupe n- ou i-propyle, un groupe méthoxy, un groupe éthoxy, un groupe méthylthio, un groupe trifluorométhyle, un groupe trifluorométhoxy, un groupe trifluorométhylthio, un groupe cyano, un groupe nitro, un groupe dioxyméthylène et un groupe dioxydifluorométhylène.

**4.** Procédé pour la préparation de 3-cyano-4-arylpyrroles selon la revendication 1, dans lequel on effectue le procédé à des températures entre -30 °C et +100 °C.

**5.** Procédé pour la préparation de 3-cyano-4-arylpyrroles selon la revendication 4, dans lequel on effectue le procédé à des températures entre -10 °C et +40 °C.

**6.** Procédé pour la préparation de 3-cyano-4-arylpyrroles selon la revendication 1, dans lequel, par mole d'amide de l'acide $\alpha$-cyanocinnamique de formule (II), on met en oeuvre de 1,0 à 2,0 moles d'isocyanure de sulfonylméthyle de formule (III) et de 1,0 à 6,0 moles de base.

**7.** Procédé pour la préparation de 3-cyano-4-arylpyrroles selon la revendication 6, dans lequel, par mole d'amide d'acide $\alpha$-cyanocinnamique de formule (II), on met en oeuvre de 1,0 à 1,3 mole d'isocyanure de sulfonylméthyle de formule (III) et de 1,0 à 3,0 moles de base.